# EUROPÄISCHE PATENTSCHRIFT

(11) **EP 1 874 678 B1**
(45) Veröffentlichungstag und Bekanntmachung des Hinweises auf die Patenterteilung: **07.09.2011**
(21) Anmeldenummer: 07700207.9
(22) Anmeldetag: 10.01.2007
(51) Int. Cl.: B81B 3/00, H04R 19/00

(54) **MEMS-SENSOR MIT DEFORMATIONSFREIER RÜCKELEKTRODE**
MEMS SENSOR COMPRISING A DEFORMATION-FREE BACK ELECTRODE
CAPTEUR MEMS COMPRENANT UNE ÉLECTRODE ARRIÈRE SANS DÉFORMATION

(30) Priorität: 11.01.2006 DE 102006001493
(43) Veröffentlichungstag der Anmeldung: 09.01.2008
(73) Patentinhaber: austriamicrosystems AG, 8141 Unterpremstätten (AT)
(72) Erfinder: SCHRANK, Franz, A-8052 Graz (AT)
(74) Vertreter: Epping - Hermann - Fischer
(86) Internationale Anmeldenummer: PCT/EP2007/000173
(87) Internationale Veröffentlichungsnummer: WO 2007/082662

(56) Entgegenhaltungen:
- DE-A1- 10 206 711
- US-A1- 2002 067 663
- US-B1- 6 698 295

## Beschreibung

MEMS-Sensoren mit kapazitiver Betriebsweise, wie sie insbesondere als Trägheitssensor, Drucksensor oder Mikrofon eingesetzt werden können, weisen üblicherweise als bewegliche Elektrode eine Membran oder eine Zunge auf, die durch die zu bestimmende physikalische Größe bewegt werden kann. In definiertem Abstand dazu ist eine Gegen- beziehungsweise Rückelektrode angeordnet.

Ein Beispiel für einen MEMS-Sensor mit kapazitiver Betriebsweise ist in der Patenschrift US 2002/0067663 A1 offenbart.

In bekannten MEMS-Sensoren bildet die Rückelektrode zusammen mit einer sie tragenden mechanisch stabilen Schicht einen den Basischip kappenförmig überragenden Aufbau aus. Ein bekanntes Material zum Aufbau der mechanisch stabilen Schicht ist dabei Siliziumnitrid. In stöchiometrischer Form kann es in einem stabilen Prozess mit konstanten Eigenschaften erzeugt werden. Nachteilig ist jedoch, dass stöchiometrisch erzeugte Siliziumnitrid eine Zugspannung erzeugt, die auf den dreidimensionalen Aufbau des MEMS-Sensors einwirkt und zu dessen Deformation und damit auch zu einer Positionsänderung der Rückelektrode führt. In deren Folge wird der Abstand zwischen beweglicher Elektrode und Rückelektrode verändert und insbesondere reduziert. Dies hat negative Folgen auf die Sensitivität des Sensors, da der in Z-Richtung deformierte Aufbau keinen definierten Abstand zwischen Rückelektrode und beweglicher Elektrode mehr aufweist. Dies macht eine exakte Messung der zu bestimmenden physikalischen Größe unmöglich.

Aufgabe der vorliegenden Erfindung ist es daher, einen MEMS-Sensor anzugeben, der diesbezügliche Probleme vermeidet.

Diese Aufgabe wird mit einem MEMS-Sensor nach Anspruch 1 gelöst. Vorteilhafte Ausgestaltungen der Erfindung sowie ein Verfahren zur Herstellung des MEMS-Sensors sind weiteren Ansprüchen zu entnehmen.

Es wird ein MEMS-Sensor mit kapazitiver Arbeitsweise angegeben, der auf einem Basischip aufgebaut ist. Er umfasst einen strukturierten Schichtaufbau, der eine zentrale Ausnehmung aufweist. Innerhalb der Ausnehmung ist in der Nähe des Basischips eine bewegliche Elektrode vorgesehen. Die Ausnehmung selbst ist mit einer auf dem Schichtaufbau aufliegenden Deckschicht überspannt, die die Rückelektrode umfasst.

Dieser Sensor hat den Vorteil, dass die Deckschicht plan auf dem Schichtaufbau aufliegt und keinen sich dreidimensional über dem Basischip oder den Schichtaufbau erhebenden Aufbau aufweist. Die bei der Herstellung der Deckschicht möglicherweise erzeugten Zugspannungen wirken daher nur in der Ebene der Deckschicht. Gleichzeitig wird die Deckschicht von dem die Ausnehmung umgebenden Schichtaufbau gehalten, sodass eine Deformation der Deckschicht und insbesondere der Rückelektrodenschicht verhindert wird. Es wird im Gegenteil beobachtet, dass innerhalb der Deckschicht auftretende Zugspannungen zu einer Stabilisierung des Schichtaufbaus führen und zu einer erhöhten Steifigkeit der Rückelektrode führen. Dies macht es möglich, dass die erforderliche mechanische Stabilität und Steifheit der die Rückelektrode umfassenden Deckschicht bereits mit geringeren Schichtdicken als bei bekannten MEMS-Sensoren erreicht werden kann.

Als weiterer Vorteil wird mit dieser Anordnung erreicht, dass für die Deckschicht auf Materialien zurückgegriffen werden kann, die bei bekannten MEMS-Sensoren eher vermieden wurden.

Beispielsweise kann für die Deckschicht nun das eingangs bereits erwähnte stöchiometrische Siliziumnitrid eingesetzt werden, welches bei der Erzeugung eine wesentliche höhere Zugspannung erzeugt als ein nicht-stöchiometrisches Siliziumnitrid mit Siliziumüberschuss. Stöchiometrisches Siliziumnitrid hat den Vorteil, dass es mechanisch stabil ist und in einem stabilen Prozess mit konstant reproduzierbaren und gleichmäßigen Schichteigenschaften erzeugt werden kann. Die unter-stöchiometrischen Siliziumnitride dagegen können nur in einem nicht-stabilen Prozess hergestellt werden, was zur Folge hat, dass der Prozess nur aufwändig kontrollierbar ist und in der Regel zu inhomogenen beziehungsweise nicht exakt reproduzierbaren Materialeigenschaften führt.

Im vorgeschlagenen MEMS-Sensor kann die Deckschicht im Bereich der Ausnehmung elektrisch leitend sein oder umfasst zumindest eine elektrisch leitende Teilschicht. Die Teilschicht kann aus Polysilizium sein, welches zur Herstellung einer ausreichenden elektrischen Leitfähigkeit hoch dotiert sein kann. Die mechanisch stabile Schicht kann dann aus Siliziumnitrid ausgebildet sein. Die Deckschicht umfasst dann zumindest zwei Schichten. Möglich ist es jedoch auch, die Deckschicht mit einer höheren Anzahl an Schichten auszuführen und insbesondere mit einem symmetrischen Schichtaufbau zu realisieren. Eine Möglichkeit besteht dann darin, in der Deckschicht zumindest eine Polysiliziumschicht vorzusehen, die zwischen zwei Teilschichten aus Siliziumnitrid eingebettet ist. In einer solchen Mehrfachschicht mit symmetrischem Aufbau können Spannungen optimal ausgeglichen werden und so ein mechanisch stabiler Mehrschichtaufbau erhalten werden, der keinerlei Deformationen zeigt.

In einem Bereich um die Ausnehmung im Schichtaufbau herum ist die Deckschicht oder zumindest eine Teilschicht der Deckschicht bis zur Oberfläche des Basischips geführt und schließt dort mit dem Basischip ab. Dementsprechend sind alle Seitenflächen der Ausnehmung vollständig beziehungsweise seitlich und oben von der Deckschicht oder einer oder mehrerer ihrer Teilschichten begrenzt. Lediglich ein Teil der beweglichen Elektrode wird durch die die Ausnehmung umgebende auf die Oberfläche des Basischips heruntergezogene Deckschicht oder Teilschicht hindurchgeleitet und außerhalb davon elektrisch kontaktiert. Direkt benachbart zu diesem Bereich, also weiter von der Ausnehmung entfernt, verläuft die Deckschicht auf der Oberfläche des Schichtaufbaus, sodass sie insgesamt eine annähernd plane Oberfläche ausbildet innerhalb der sich die Zugspannungen optimal ausgleichen lassen.

Die bewegliche Elektrode kann als Zunge ausgebildet sein, die im lichten Abstand zur Oberfläche des Basischips angeordnet und beispielsweise mit einem Ende im Schichtaufbau fixiert ist. Möglich ist es jedoch auch, die bewegliche Elektrode als Membran auszubilden, die direkt auf der Oberfläche des Basischips aufliegt. In diesem Fall ist unterhalb der Membran eine Durchbrechung im Basischip vorgesehen, die von der Membran abgedeckt wird. So ist es möglich, die Membran zur Detektion von Druckunterschieden oder als Mikrofon zu nutzen.

Die Elektrodeneigenschaften der beweglichen Elektrode beziehungsweise der Membran können durch leitfähig eingestelltes Polysilizium erzielt werden. Dabei ist es möglich, die bewegliche Elektrode entweder vollständig aus Polysilizium auszubilden, oder als Kombination einer Polysiliziumschicht und einer oder mehreren mechanisch stabilen Trägerschichten oder anderen Schichten gegebenenfalls mit symmetrischem Aufbau zu realisieren.

Ein als Mikrofon oder Drucksensor ausgebildeter MEMS-Sensor kann in der Deckschicht im Bereich der Ausnehmung ein Muster von Öffnungen aufweisen, sodass die bewegliche Elektrode durch die Deckschicht zwar von mechanischen Einflüssen geschützt, jedoch mit dem Umgebungsdruck durch die Öffnungen hindurch in Verbindung steht.

Eine als Membran ausgebildete bewegliche Elektrode liegt zwar frei auf der Oberfläche des Basischips über der Durchbrechung auf, wird jedoch vorzugsweise in dieser Position fixiert und kann mit Membranhaltern gegen den Basischip angedrückt sein. Dazu können in der Deckschicht im Randbereich der Membran Ausstülpungen in die Ausnehmung hinein vorgesehen sein, die bis unmittelbar über die Oberfläche der Membran reichen und somit deren Bewegungsspielraum in Richtung Rückelektrode beziehungsweise Deckschicht minimieren. Diese Fixierung der Membran ausschließlich in deren Randbereich garantiert, dass sie im zentralen Bereich ungehindert auslenken kann.

Eine Auslenkung der Membran bis zur Unterkante der Deckschicht kann sowohl beim Herstellungsprozess als auch während des Betriebs auftreten und zu einem Verhaken oder gar Verschweißen der Membran mit der Deckschicht führen, insbesondere wenn die Unterseite der Deckschicht und die Oberseite der Membran aus gleichem Material und insbesondere aus Polysilizium ausgebildet sind. Dies würde zu einer Blockade der Membran und damit zu einer Fehlfunktion des MEMS-Sensors führen. Um dies zu verhindern, können in der Deckschicht Abstandshalter in Form von nach innen weisenden Ausstülpungen vorgesehen sein, die in der Deckschicht im Bereich oberhalb der Membran regelmäßig verteilt sind. Um ein Verbacken der beiden Materialien zu vermeiden, bestehen diese Ausstülpungen nicht aus Polysilizium, sondern vorzugsweise aus Siliziumnitrid.

Der Schichtaufbau umfasst Opferschichten, Elektrodenschichten und gegebenenfalls Strukturmaterialschichten, die bei der Herstellung vorzugsweise jeweils ganzflächig aufgebracht werden. Elektroden- und Strukturmaterialschichten können nach dem ganzflächigen Aufbringen jeweils in gewünschter Weise strukturiert oder auch planarisiert werden. Die Verwendung von Opfermaterial dient insbesondere dazu, um nach vollständiger Herstellung des Schichtaufbaus die Ausnehmung durch Herauslösen oder -Ätzen von Opferschichtmaterial zu erzeugen. Ein geeignetes Material für eine Opferschicht umfasst Siliziumoxid, welches sich gegenüber vielen anderen Materialien spezifisch ätzen lässt. Dotierte Glasschichten lassen sich besonders einfach nasschemisch herauslösen.

Im Folgenden wird die Erfindung anhand von Ausführungsbeispielen und den dazugehörigen Figuren näher erläutert. Die Figuren dienen allein der Veranschaulichung der Erfindung und sind daher nur schematisch und nicht maßstabsgetreu ausgeführt.

Es zeigen:
Figuren 1 bis 10 verschiedene Verfahrensstufen gemäß einer ersten Herstellungsvariante,
Figuren 11 bis 15 verschieden Verfahrensstufen gemäß einer weiteren Herstellungsvariante, und
Figur 16 eine Darstellung der in einem MEMS-Sensor auftretenden Zug- und Druckspannungen im Vergleich zu bekannten Sensoren.

Im Folgenden wird die Herstellung eines als Mikrofon ausgebildeten MEMS-Sensors beschrieben. Grundlage für den MEMS-Sensor ist ein Basischip BC, der zumindest eine mechanische Trägerfunktion erfüllt. Er ist daher aus einem mechanisch stabilen und insbesondere verzugsfesten Material ausgebildet, vorzugsweise aus einem kristallinen Material und besteht beispielsweise aus einem Siliziumwafer. Dementsprechend wird ein erfindungsgemäßer MEMS-Sensor auch nicht als Einzelbauelement sondern auf Waferebene gefertigt, wobei eine Vielzahl von MEMS-Sensor parallel auf der Waferoberfläche prozessiert werden.

Zunächst wird ein Schichtaufbau SA erzeugt, der eine Mehrzahl von Schichtabscheidungs- und Strukturierungsschritten umfasst. Im vorliegenden Ausführungsbeispiel wird dazu zumindest eine erste Opferschicht OS, darüber eine erste Polysiliziumschicht, aus der die Membran ME strukturiert wird, und darüber eine zweite Opferschicht erzeugt. Wahlweise kann anschließend ein Planarisierungsprozess durchgeführt werden, zum Beispiel ein CMP-Prozess (chemical mechanical polishing) um eine plane Oberfläche für die weiteren Schichterzeugungsprozesse und insbesondere für die Deckschicht zu erhalten.

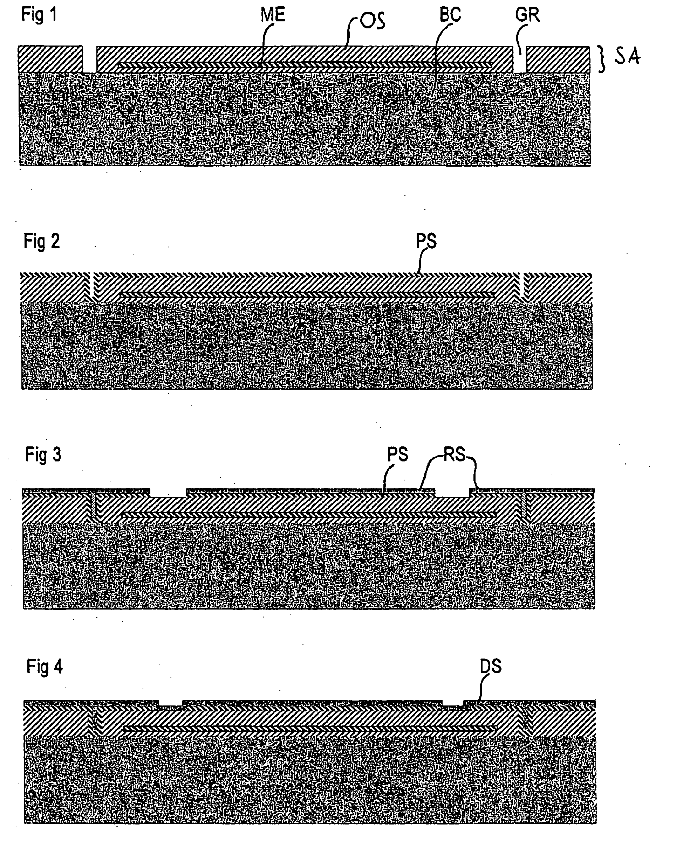

Figur 1 zeigt anhand eines schematischen Querschnitts die Anordnung im Bereich eines einzelnen MEMS-Sensors, bei der im Schichtaufbau SA bereits ein Graben GR rund um die Membran ME herum erzeugt ist, in dem die Oberfläche des Basischips BC freigelegt ist. Die Membran ME hat eine beliebige, vorzugsweise eine runde oder rechteckige Grundfläche und setzt sich mit einer schmalen Zuleitung in den Schichtaufbau SA fort (in der Figur nicht dargestellt). Dementsprechend ist auch der Graben GR in Abhängigkeit von der Membrangrundfläche als geschlossener Kreis oder geschlossenes Rechteck rund um die Membran herum geführt.

Im nächsten Schritt wird eine zweite Polysiliziumschicht PS kantenbedeckend so auf der Oberfläche abgeschieden, dass auch der Graben GR an Seitenwänden und Boden von der Polysiliziumschicht ausgekleidet ist. Figur 2 zeigt die Anordnung auf dieser Verfahrensstufe.

Die Polysiliziumschicht PS kann hochdotiert erzeugt werden oder nachträglich mit einem geeigneten Verfahren dotiert werden, wie dies beispielsweise anhand von Figur 4 später noch exemplarisch dargestellt ist.

Figur 3 zeigt die Strukturierung der zweiten Polysiliziumschicht PS, die mittels einer ebenfalls strukturierten Resistschicht RS1 durchgeführt wird. Zur Strukturierung können beliebige zum Ätzen von Silizium geeignete Verfahren eingesetzt werden, beispielsweise reaktives Ionenätzen.

Figur 4 zeigt eine dielektrische Hilfsschicht, vorzugsweise eine weitere Opferschicht, die nach dem Entfernen der Resistschicht RS1 kantenbedeckend auf der gesamten Oberfläche aufgebracht wird. In einer Verfahrensvariante kann diese Opferschicht DS eine hochdotierte Siliziumoxidschicht sein, mit deren Hilfe nachträglich Dotierstoff in die zweite Polysiliziumschicht PS in einem Anneal-Schritt eingetrieben werden kann. In diesem Fall ist die Breite des durch die erste Polysiliziumschicht PS verengten Grabens ausreichend gewählt, um eine Befüllung mit dieser dielektrischen Opferschicht zu ermöglichen. In einem Ausführungsbeispiel besteht die dielektrische Opferschicht DS aus Phosphorsilikatglas.

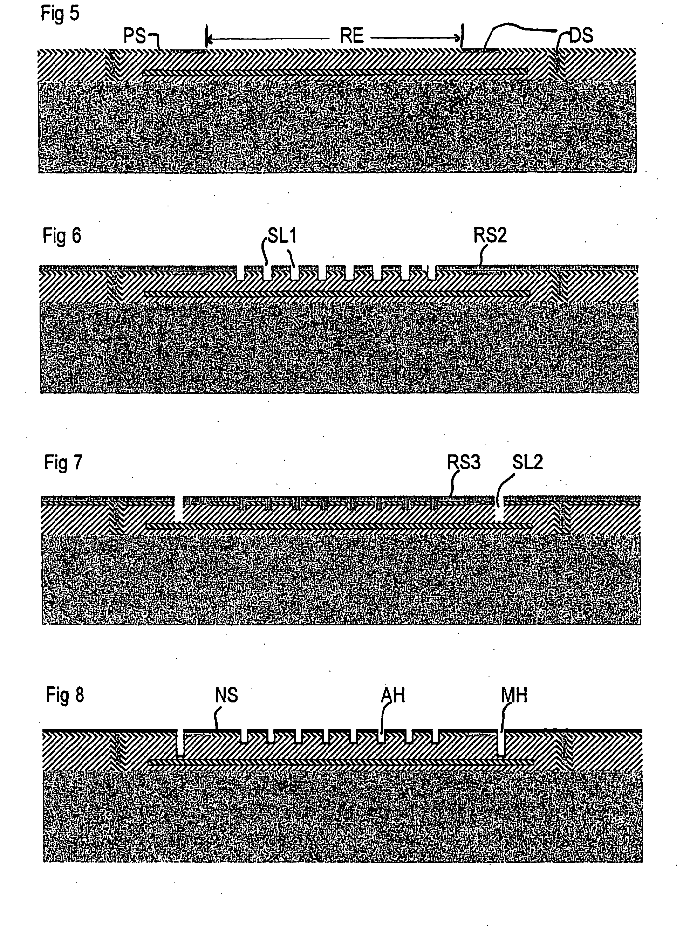

Figur 5 zeigt die Anordnung nach Planarisieren der Oberfläche, was zum Beispiel wiederum mittels eines CMP-Verfahrens erfolgen kann. Dabei wird die Oberfläche der zweiten Polysiliziumschicht PS freigelegt. Der Strukturierungsgraben der zweiten piezoelektrischen Schicht, der die Rückelektrode von dem übrigen Bereich der Polysiliziumschicht trennt, ist nun ebenso wie der gegebenenfalls verbleibende Graben GR mit der dielektrischen Opferschicht DS gefüllt.

In dem in Figur 6 gezeigten Schritt wird nun in die Oberfläche der Polysiliziumschicht bis in die Opferschicht des Schichtaufbaus hineinreichend ein Muster von Öffnungen in Form von Sacklöchern SL1 erzeugt, die im Bereich der aus der Polysiliziumschicht PS strukturierten Rückelektrode gleichmäßig verteilt sind.

Es wird dazu eine strukturierte Resistschicht RS2 verwendet und ein geeignetes Ätzverfahren für Polysilizium beziehungsweise für die darunter liegende Opferschicht eingesetzt.

Im nächsten Schritt werden weitere Sacklöcher SL2 zur Herstellung der Membranhalterung geätzt. Dazu wird eine weitere Resistschicht RS3 eingesetzt, die außerhalb der zur Rückelektrode RE strukturierten zweiten Polysiliziumschicht PS vorgesehen werden.

Die weiteren Sacklöcher SL2 werden bis knapp über die Oberfläche der Membran ME geätzt. Bezüglich der 2D-Anordnung sind die weiteren Sacklöcher SL2 vorzugsweise gleichmäßig über den Randbereich der Membran ME verteilt.

Nach Entfernen der Resistschicht RS3 wird eine mechanisch stabile Teilschicht der Deckschicht abgeschieden, wozu insbesondere stöchiometrisches Siliziumnitrid verwendet wird. Alternativ zu dem Siliziumnitrid kann auch jedes andere mechanisch stabile Material verwendet werden, welches sich spezifisch zum Opfermaterial und insbesondere spezifisch zu dem dafür verwendeten Siliziumoxid ätzen lässt.

Die Abscheidung erfolgt kantenbedeckend, sodass wie in Figur 8 dargestellt sowohl die ersten Sacklöcher als auch die zweiten Sacklöcher an Seitenwänden und Böden mit der Siliziumnitridschicht NS ausgekleidet sind. Diese Schicht bildet nun in den ersten Sacklöchern die Abstandshalter AH aus, in den zweiten Sacklöchern dagegen die Membranhalter MH. Abstandshalter AH und Membranhalter MH unterscheiden sich insbesondere durch die in den Schichtaufbau SA hineinreichende Tiefe und können ansonsten gleich ausgebildet sein. Daher ist auch die Reihenfolge bei der Herstellung der entsprechenden Sacklöcher austauschbar. Alternativ zur oben beschriebenen Herstellungsvariante können die zweiten Sacklöcher auch vor den ersten Sacklöchern in voneinander unterschiedlichen Strukturierungsschritten hergestellt werden. Beide Fälle führen zu dem in Figur 8 dargestellten Aufbau.

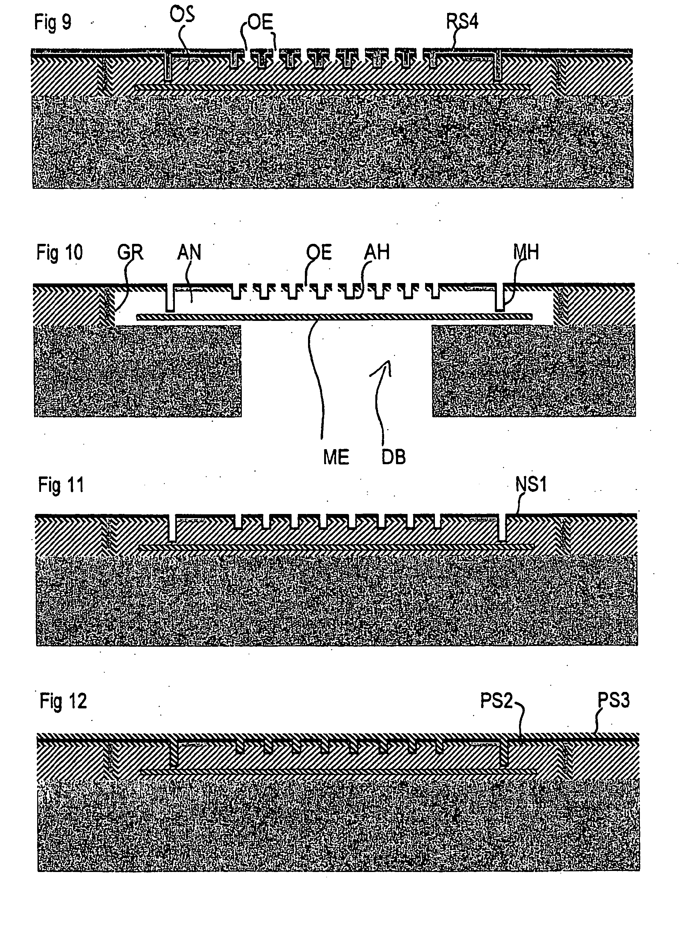

Im nächsten in Figur 9 dargestellten Verfahrensschritt wird nun im Bereich der Rückelektrode RE ein Muster von Öffnungen OE durch die aus zweiter Polysiliziumschicht PS und Siliziumnitridschicht NS bestehende Deckschicht geätzt. Die Öffnungen sind dabei zwischen den Abstandshaltern angeordnet. Die Öffnungen OE werden vorzugsweise ebenfalls in Form von Sacklöchern mit beliebigem z.B. runden oder rechteckigen Querschnitt hergestellt. Die Tiefe der Sacklöcher ist hierbei unkritisch, sodass ein beliebiges gegebenenfalls auch unspezifisches Ätzverfahren eingesetzt werden kann. Zur Strukturierung dient hier eine weitere Resistschicht RS4.

Im nächsten Schritt wird das Material der Opferschichten im Bereich von Membran ME und Rückelektrode RE entfernt. Der Bereich zwischen Membran ME und Deckschicht ist dabei durch die Öffnungen OE zugänglich. Aufgrund des relativ geringen Abstandes zwischen Membran ME und Oberfläche des Basischips wird das Material der Opferschicht unterhalb der Membran jedoch vorzugsweise von der Seite des Basischips BC her geätzt. Dazu wird im Basischip BC von der Rück- oder Unterseite her eine Durchbrechung DB erzeugt, in der die Oberfläche der untersten Opferschicht freigelegt wird. Die Durchbrechung entspricht im Querschnitt ungefähr der Größe der Rückelektrode, weist auf jeden Fall aber eine kleinere Querschnittsfläche als die Membran ME auf, sodass die Membran in einem Randbereich rund um die Durchbrechung DB auf dem Basischip aufliegen kann.

Die Durchbrechung DB kann mit anisotropen Ätzverfahren erzeugt werden, wobei die dargestellten annähernd vertikalen Seitenwände erhalten werden. Es ist jedoch auch möglich, ein kristallachsenspezifisches Ätzverfahren einzusetzen, wobei in einem kristallinen Material des Basischips wie beispielsweise in Silizium auch schräge Seitenwände erhalten werden. Der Angriff des Ätzmittels ist nun von oben und unten möglich, sodass nach Herauslösen der untersten Opferschicht die Membran ME nun frei auf dem Basischip aufliegt und keine feste Verbindung mehr zum Basischip aufweist. Auch die oberhalb der Membran ME liegenden Opferschichten werden vollständig herausgelöst, wobei sich die Ausnehmung AN ergibt, die vollständig von dem mit Polysilizium gefüllten Graben GR begrenzt ist.

Zum Ätzen des Opfermaterials wird ein gegen Siliziumnitrid und Polysilizium spezifisches Ätzverfahren eingesetzt, so dass das Polysilizium im Graben GR als Ätzstopp dienen kann.

Figur 10 zeigt den so fertig gestellten MEMS-Sensor mit einer frei schwingbaren Membran ME, ausgebildet aus elektrisch leitfähigem Polysilizium, und der Rückelektrode RE, die als elektrisch leitende Teilschicht aus Polysilizium auf der mechanisch stabilen Siliziumnitridschicht aufgebracht und mit dieser zusammen die Deckschicht ausbildet. Die Membranhalter MH fixieren die Membran ME im Randbereich auf der Oberfläche des Basischips während die Abstandshalter AH in ausreichendem Abstand zur Membran den direkten Kontakt der Membran ME mit der zweiten Polysiliziumschicht PS der Deckschicht bei zu starker Auslenkung der Membran ME verhindern.

Außerhalb der vom Graben GR umschlossenen Ausnehmung AN ist der Schichtaufbau SA unversehrt beziehungsweise nicht durch einen der Ätzschritte angegriffen. Er umgibt stabilisierend die Ausnehmung und gibt das Niveau für die darauf aufliegende nach außen beziehungsweise oben annähernd plane Deckschicht vor. Die aus Siliziumnitridschicht und zweiter Polysiliziumschicht bestehende Deckschicht ist mit Ausnahme der nur punktförmig platzierten Abstandshalter und Membranhalter ebenfalls plan und mit annähernd konformer Schichtdicke ausgebildet. Zug- und Druckspannungen können sich daher gleichmäßig verteilen und werden durch Abstützung auf dem verbleibenden Schichtaufbau SA kompensiert, sodass es zu keinerlei Verbiegungen der Deckschicht und insbesondere der Rückelektrode in Richtung Membran ME kommt. Die Verspannung der Deckschicht mit dem verbleibenden Schichtaufbau SA führt dazu, dass die mechanische Stabilität gewährende Siliziumnitridschicht dünner als bisher bekannt ausgeführt werden kann. Dennoch wird eine ausreichende Steifheit der Deckschicht erzielt. Während bei bekannten MEMS-Mikrofonen beispielsweise ein nicht-stöchiometrisches Siliziumnitrid mit Siliziumüberschuss in einer Schichtdicke von circa 1,5 µm eingesetzt wurde, welches einen Stress von circa 100 MPa erzeugt, kann im Vergleich dazu nun ein stöchiometrisches Standardsiliziumnitrid in einer Schichtdicke von circa 0,5 µm eingesetzt werden, welches zwar einen Stress von circa 1,3 GPa erzeugt, der mit dem vorgeschlagenen Aufbau jedoch unschädlich ohne Verzug der Deckschicht kompensiert werden kann. Die dünnere Materialschicht ermöglicht eine Material- und Zeiteinsparung während Herstellung und Strukturierung. Ein erfindungsgemäßer MEMS-Sensor ist daher auch kostengünstiger zu realisieren.

Eine Verfahrensvariante zur Herstellung eines MEMS-Sensors mit symmetrisch aufgebauter Deckschicht ist anhand der Figuren 11 bis 15 erläutert. Ausgehend von der in Figur 11 dargestellten Verfahrensstufe nach dem Abscheiden der Polysiliziumschicht PS (entspricht der Figur 8), wird im nächsten Schritt zunächst über der Siliziumnitridschicht eine weitere Polysiliziumschicht PS3 kantenbedeckend aufgebracht und gegebenenfalls anschließend planarisiert. Figur 12 zeigt die Anordnung auf dieser Verfahrensstufe. Über der planen Oberfläche wird nun eine zweite Siliziumnitridschicht NS2 erzeugt, vorzugsweise in der gleichen Schichtdicke wie die erste Siliziumnitridschicht NS1.

Ähnlich wie in Figur 9 werden nun in diese Dreifachschicht, die durch die darunter liegende zweite Polysiliziumschicht PS2 sogar eine Vierfachschicht ist, mit Hilfe einer Resistschicht RS4 Öffnungen OE geätzt. Die Tiefe der Öffnungen wird so bemessen, dass an deren Boden die Oberfläche der Opferschicht freigelegt ist. Auch hier ist ein zu tiefes Ätzen, sofern es im Bereich der Opferschicht OS bleibt, unschädlich.

Ähnlich wie anhand von Figur 10 erläutert wird nun auch hier an dem Basischip BC eine Durchbrechung DB unterhalb der Membran ME erzeugt und anschließend das Opfermaterial im Bereich der Ausnehmung herausgelöst. Auch hier kann dazu ein geeignetes selektives Ätzverfahren eingesetzt werden. Wird als Opferschicht eine hochdotierte Siliziumoxidschicht und beispielsweise Phosphorsilikatglas verwendet, so kann ein nasschemisches Ätzverfahren auf Flusssäurebasis durchgeführt werden. Möglich ist es jedoch auch, das Ätzverfahren mit gasförmigem Fluorwasserstoffgas durchzuführen. Dabei ist es vorteilhaft, das Material der Opferschichten nicht zu hoch zu dotieren, um bei der Gasphasenätzung keine festen Nebenprodukte des Fluorwasserstoffgases mit den Dotierstoffen zu erhalten, die unerwünschte Rückstände im Bereich der Ausnehmung ausbilden könnten.

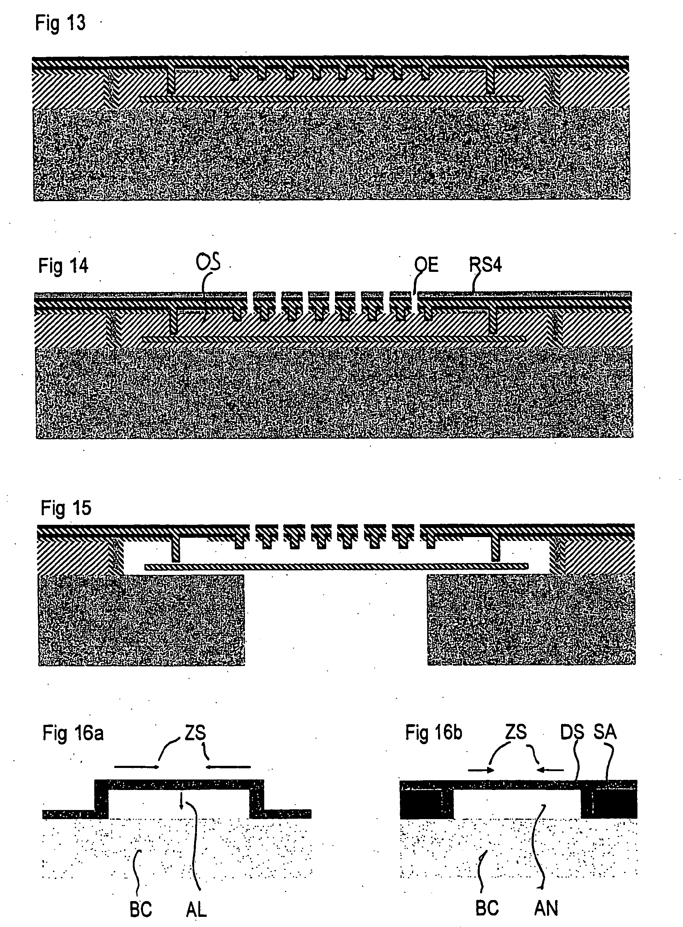

Figur 15 zeigt die nach diesem Ätzschritt fertiggestellte Struktur eines MEMS-Sensors mit einer Deckschicht, die eine Siliziumnitrid/Polysilizium/Siliziumnitriddreifachschicht umfasst.

Die in den Figuren nicht dargestellten elektrischen Anschlüsse des MEMS-Sensors werden bei den Strukturierungsschritten für die Polysiliziumschicht der Membran ME sowie der die Rückelektrode RE bildenden zweiten Polysiliziumschicht PS mit strukturiert. Es werden dabei insbesondere elektrische Zuleitungen geschaffen, die elektrisch mit entsprechenden Anschlussflächen verbunden sind. Diese Anschlussflächen können auf der Oberfläche des Schichtaufbaus erzeugt werden. Möglich ist es jedoch auch, die Anschlüsse teilweise oder vollständig auf der Oberfläche des Basischips anzuordnen. Dazu wird außerhalb des in den Figuren dargestellten Sensorbereichs der Schichtaufbau stellenweise so weit entfernt, dass die Oberfläche des Basischips freigelegt wird. Dort werden die entsprechenden elektrischen Anschlussflächen, insbesondere bondbare Oberflächen erzeugt und in geeigneter Weise mit zweiter Polysiliziumschicht PS und Membran ME verbunden.

Während das Herstellungsverfahren auf Waferebene parallel für eine Vielzahl von Einzelsensoren durchgeführt wird, kann die Vereinzelung in einzelne Sensorelemente in einem letzten Schritt erfolgen, wobei zur Auftrennung beispielsweise ein Sägeverfahren eingesetzt werden kann.

Figur 16 zeigt nochmals die Vorteile beziehungsweise vorteilhaften Wirkungen des erfindungsgemäßen Aufbaus im Vergleich zu bekannten Sensoraufbauten. Figur 16A zeigt schematisch eine bekannte auf einem Basischip aufsitzende Deckschicht, die im Bereich des Sensor einen 3D-Aufbau aufweist und sich über die Oberfläche des Basischips erhebt beziehungsweise nach außen wölbt. Die herstellungsbedingten und insbesondere bei der Abscheidung der Siliziumnitridschicht erzeugten Zugspannungen ZS sind durch zwei waagrecht angeordnete Pfeile verdeutlicht. Diese führen dazu, dass sich der dreidimensionale Aufbau der Deckschicht bei bekannten Sensoren verziehen kann, was in einer Auslenkung AL der Deckschicht hin zur Oberfläche des Basischips BC beziehungsweise der dort angeordneten beweglichen Elektrode führt. Diese Auslenkung ist durch Pfeile AL angedeutet.

Figur 16B dagegen zeigt schematisch den Aufbau eines erfindungsgemäßen MEMS-Sensors, bei dem die annähernd durchgehend plan ausgebildete Deckschicht DS allseitig auf einem verbleibenden Schichtaufbau SA aufliegt. Die auch hier auftretenden Zugspannungen ZS werden dadurch kompensiert und führen zu keinerlei Auslenkung der Deckschicht im Bereich der Ausnehmung AN. Dadurch bleiben die Sensordimensionen konstant, insbesondere der Abstand zwischen Rückelektrode RE und beweglicher Elektrode, beispielsweise Membran ME. Ein sicher einstellbarer Abstand zwischen Rückelektrode und Membran ermöglicht eine exakte Messung der beobachteten physikalischen Größe, beispielsweise des auf die Membran einwirkenden Schalldrucks.

Die Erfindung ist nicht auf die dargestellten Ausführungsbeispiele beschränkt. Die genaue Struktur und der genaue Aufbau erfindungsgemäßer MEMS-Sensoren wird allein durch die Ansprüche definiert und kann von den dargestellten Ausführungsformen abweichen.

### Bezugszeichenliste

- BC: Basischip
- SA: Schichtaufbau
- AN: Ausnehmung
- GR: Graben
- ME: bewegliche Elektrode
- DS: Deckschicht
- DB: Durchbrechung
- RE: Rückelektrode
- OS: Opferschicht
- NS: Siliziumnitridschicht
- PS: Polysiliziumschicht
- RS: Resistschicht
- AH: Abstandshalter
- MH: Membranhalter
- SL: Sacklöcher
- OE: Öffnungen
- ZS: Zugspannung
- AL: Auslenkung

## Patentansprüche

1. MEMS-Sensor mit kapazitiver Arbeitsweise
- mit einem Basischip (BC)
- mit einem auf der Oberfläche des Basischips aufgebrachten strukturierten Schichtaufbau (SA)
- mit einer Ausnehmung (AN) im Schichtaufbau, die von einem Randbereich des Schichtaufbaus umgeben ist
- mit einer in der Ausnehmung angeordneten und mit dem Schichtaufbau verbundenen beweglichen Elektrode (ME)
- mit einer eine Rückelektrode (RE) umfassenden Deckschicht (DS), die um die Ausnehmung herum auf dem Schichtaufbau aufliegt und die Ausnehmung überspannt
- bei dem die Deckschicht (DS) oder eine Teilschicht der Deckschicht in einem die Ausnehmung (AN) umschließenden Bereich innerhalb des Schichtaufbau (SA) bis zur Oberfläche des Basischips (BC) geführt ist, dort mit dem Basischip abschließt und dabei die Ausnehmung seitlich rundum begrenzt.

2. MEMS-Sensor nach Anspruch 1,
bei dem die Deckschicht (DS) im Bereich der Ausnehmung (AN) eine elektrisch leitende Teilschicht umfasst.

3. MEMS-Sensor nach Anspruch 2,
bei dem die Deckschicht (DS) eine Teilschicht aus Polysilizium umfasst.

4. MEMS-Sensor nach einem der Ansprüche 1 bis 3,
bei dem die Oberseite der Deckschicht (DS) im Bereich der Ausnehmung (AN) und dem sie umgebenden Randbereich auf dem gleichen Niveau über der Oberfläche des Basischips (BC) liegt.

5. MEMS-Sensor nach einem der Ansprüche 2 bis 4,
bei dem die elektrisch leitende Schicht oder Teilschicht der beweglichen Elektrode (ME) und/oder der Deckschicht (DS) aus Polysilizium ausgebildet ist.

6. MEMS-Sensor nach einem der Ansprüche 1 bis 5,
bei dem die Deckschicht (DS) im Bereich der Ausnehmung (AN) ein Muster von Öffnungen (OE) aufweist.

7. MEMS-Sensor nach einem der Ansprüche 1 bis 6,
bei dem die Deckschicht (DS) eine Siliziumnitridschicht umfasst.

8. MEMS-Sensor nach Anspruch 7,
bei dem die Deckschicht (DS) zwei Siliziumnitridschichten umfasst, zwischen denen eine Polysiliziumschicht (PS) angeordnet ist.

9. MEMS-Sensor nach einem der Ansprüche 1 bis 8,
bei dem die bewegliche Elektrode (ME) als Membran ausgebildet ist und die Deckschicht (DS) oder eine Teilschicht der Deckschicht im Randbereich aber innerhalb der Ausnehmung (AN) Ausstülpungen in Richtung Basischip (BC) aufweist, die bis unmittelbar über die Membran reichen.

10. MEMS-Sensor nach einem der Ansprüche 1 bis 9,
bei dem die Membran über einer durch die gesamte Dicke des Basischips (BC) führenden Durchbrechung (DB) angeordnet ist.

11. MEMS-Sensor nach einem der Ansprüche 1 bis 9,
bei dem das Opferschichtmaterial Siliziumoxid umfasst.

12. Verfahren zur Herstellung eines MEMS-Sensors mit den Verfahrensschritten
A. Aufbringen eines Schichtaufbaus (SA) mit planer Oberfläche auf der Oberfläche eines Basischips (BC), umfassend eine Reihe von Schichterzeugungs - und Schichtstrukturierungsschritten, wobei der Schichtaufbau eine zentral über dem Basischip angeordnete bewegliche Elektrode (ME) umfasst, die zwischen zwei Opferschichten (OS) angeordnet ist
B. Ätzen eines Grabens (GR) in den Schichtaufbau, der die bewegliche Elektrode im Abstand umschließt und bis zur Oberfläche des Basischips reicht
C. Aufbringen einer Deckschicht (DS) auf der gesamten Oberfläche des Schichtaufbaus und im Graben
D. Erzeugen eines Musters von Öffnungen (OE) in der Deckschicht über der beweglichen Elektrode
E. Entfernen der Opferschichten (OE) über und unter der beweglichen Elektrode durch Ätzen, wobei die den Graben (GR) zumindest auskleidende Deckschicht (DS) als Ätzstop dient, wobei eine von einem ringförmigen Randbereich des Schichtaufbaus umgebene Ausnehmung (AN) entsteht, die von der Deckschicht überspannt wird und in der die bewegliche Elektrode angeordnet ist.

13. Verfahren nach Anspruch 12,
bei dem die Opferschichten (OS) Siliziumoxid umfassen und bei dem ein fluorwasserstoffhaltiges Ätzmittel zur Entfernung der Opferschichten eingesetzt wird.

14. Verfahren nach Anspruch 12 oder 13,
bei dem zur Erzeugung der Deckschicht (DS) im Schritt C zumindest die folgenden Teilschritte eingesetzt werden
C1) Abscheiden einer kantenbedeckenden zweiten Polysiliziumschicht (PS2)
C2) Strukturieren der zweiten Polysiliziumschicht zu einer Rückelektrode
C3) Abscheiden und Planarisieren einer Oxidschicht C4) Abscheiden einer ersten Siliziumnitridschicht (NS).

15. Verfahren nach einem der Ansprüche 12 bis 14,
bei dem im Verfahrensschritt A folgende Teilschritte durchgeführt werden
A1) Erzeugen zumindest einer unteren Opferschicht (OS),
A2) Erzeugen einer dotierten ersten Polysiliziumschicht (PS) darüber
A3) Strukturieren der ersten Polysiliziumschicht zu einer beweglichen Elektrode (ME)
A4) Erzeugen zumindest einer oberen Opferschicht (OS)
A5) Planarisieren des Schichtaufbaus (SA).

16. Verfahren nach Anspruch 15,
- bei dem in Schritt A3) erste Polysiliziumschicht (PS1) zu einer Membran strukturiert wird
- bei dem anschließend in die zweite Polysiliziumschicht (PS2) bis in die obere Opferschicht reichende Sacklöcher (SL) und bis zur Membran reichende weitere Sacklöcher geätzt werden,
- bei dem in Schritt C4) die Siliziumnitridschicht (NS) kantenbedeckend damit auch in allen Sacklöchern abgeschieden wird.

17. Verfahren nach einem der Ansprüche 14 bis 16,
bei dem nach Schritt C3) in einem Schritt C4) eine weitere Polysiliziumschicht (PS) über der Siliziumnitridschicht erzeugt wird,
bei der darüber in einem Schritt C5) eine weitere Siliziumnitridschicht (NS) erzeugt wird.

18. Verfahren nach einem der Ansprüche 12 bis 17,
bei dem vor Schritt E) in den Basischip (BC) von der dem Schichtaufbau (SA) gegenüberliegenden Rückseite her eine Durchbrechung (DB) geätzt wird, in der die untere Opferschicht (OS) unterhalb der als Membran ausgebildeten beweglichen Elektrode (ME) freigelegt wird.

## Claims

1. An MEMS sensor with capacitive operation
- comprising a base chip (BC)
- comprising a structured layer system (SA) applied to the surface of the base chip
- comprising a recess (AN) in the layer system surrounded by a border area of the layer system
- comprising a movable electrode (ME) arranged in the recess and connected to the layer system
- comprising a covering layer (DS) which includes a rear electrode (RE), rests on the layer system all around the recess and spans the recess
- in which the covering layer (DS) or a partial layer of the covering layer extends as far as to the surface of the base chip (BC) in a region within the layer system (SA) surrounding the recess (AN), ends there together with the base chip and laterally delimits the recess all around.

2. The MEMS sensor according to claim 1,
in which the covering layer (DS) comprises an electrically conductive partial layer in the region of the recess (AN).

3. The MEMS sensor according to claim 2,
in which the covering layer (DS) comprises a partial layer made of polysilicon.

4. The MEMS sensor according to any of the claims 1 to 3,
in which the upper side of the covering layer (DS) in the region of the recess (AN) and in the border area surrounding it lies at the same level above the surface of the base chip (BC).

5. The MEMS sensor according to any of the claims 2 to 4,
in which the electrically conductive layer or partial layer of the movable electrode (ME) and/or of the covering layer (DS) is made from polysilicon.

6. The MEMS sensor according to any of the claims 1 to 5,
in which the covering layer (DS) comprises a pattern of openings (OE) in the region of the recess (AN).

7. The MEMS sensor according to any of the claims 1 to 6,
in which the covering layer (DS) comprises a silicon nitride layer.

8. The MEMS sensor according to claim 7,
in which the covering layer (DS) comprises two silicon nitride layers between which a polysilicon layer (PS) is arranged.

9. The MEMS sensor according to any of the claims 1 to 8,
in which the movable electrode (ME) is formed as a membrane and the covering layer (DS) or a partial layer of the covering layer comprises, in the border area but within the recess (AN), protuberances towards the base chip (BC) reaching immediately above the membrane.

10. The MEMS sensor according to any of the claims 1 to 9,
in which the membrane is arranged above a through-hole (DB) extending through the entire thickness of the base chip (BC).

11. The MEMS sensor according to any of the claims 1 to 9,
in which the sacrificial layer material comprises silicon oxide.

12. A method of manufacturing an MEMS sensor, comprising the procedural steps
A. Applying a layer system (SA) with planar surface on the surface of a base chip (BC), comprising a series of layer production and layer structuring steps, wherein the layer system comprises a movable electrode (ME) centrally arranged above the base chip and arranged between two sacrificial layers (OS)
B. Etching a trench (GR) into the layer system, said trench surrounding the movable electrode so as to be spaced from it and reaching up to the surface of the base chip
C. Applying a covering layer (DS) on the entire surface of the layer system and in the trench
D. Producing a pattern of openings (OE) in the covering layer above the movable electrode
E. Removing the sacrificial layers (OE) above and below the movable electrode by means of etching, the covering layer (DS) at least lining the trench (GR) serving as an etch stopper, wherein a recess (AN) is formed which is surrounded by an annular border area of the layer system, is spanned by the covering layer and in which the movable electrode is arranged.

13. The method according to claim 12,
in which the sacrificial layers (OS) comprise silicon oxide and in which a hydrogen fluoride containing etching agent is used for removing the sacrificial layers.

14. The method according to claim 12 or 13,
in which, for producing the covering layer (DS) in step C, at least the following sub-steps are used
C1) Depositing an edge-covering, second polysilicon layer (PS2)
C2) Structuring the second polysilicon layer to form a rear electrode
C3) Depositing and planarizing an oxide layer C4) Depositing a first silicon nitride layer (NS).

15. The method according to any of the claims 12 to 14,
in which in procedural step A the following sub-steps are carried out
A1) Producing at least one lower sacrificial layer (OS),
A2) Producing a doped first polysilicon layer (PS) thereon
A3) Structuring the first polysilicon layer to form a movable electrode (ME)
A4) Producing at least one upper sacrificial layer (OS)
A5) Planarizing the layer system (SA).

16. The method according to claim 15,
- in which in step A3) the first polysilicon layer (PS1) is structured to form a membrane
- in which, subsequently, blind holes (SL) reaching into the upper sacrificial layer and additional blind holes reaching to the membrane are etched into the second polysilicon layer (PS2),
- in which in step C4) the silicon nitride layer (NS) is deposited so as to cover all edges and consequently also deposited in all blind holes.

17. The method according to any of the claims 14 to 16,
in which, after step C3) and in a step C4), a further polysilicon layer (PS) is produced over the silicon nitride layer,
in which a further silicon nitride layer (NS) is produced thereon in a step C5).

18. The method according to any of the claims 12 to 17,
in which, prior to step E), a through-hole (DB) is etched into the base chip (BC) starting from the rear side opposite the layer system (SA), in which through-hole the lower sacrificial layer (OS) is exposed underneath the movable electrode (ME) formed as a membrane.

## Revendications

1. Capteur MEMS à fonctionnement capacitif
- avec une puce de base (BC)
- avec un système de couches (SA) structuré appliqué sur la surface de la puce de base
- avec une cavité (AN) dans le système de couches, qui est entourée par une zone limitrophe du système de couches
- avec une électrode mobile (ME) disposée dans la cavité et reliée au système de couches
- avec une couche de recouvrement (DS) comprenant une électrode arrière (RE), reposant sur le système de couches tout autour de la cavité et enjambant la cavité
- où la couche de recouvrement (DS) ou une couche partielle de la couche de recouvrement est menée, dans une zone entourant la cavité (AN) à l'intérieur du système de couches (SA), jusqu'à la surface de la puce de base (BC), y vient en contact avec la puce de base et délimite ce faisant la cavité latéralement sur toute la périphérie.

2. Capteur MEMS selon la revendication 1,
où la couche de recouvrement (DS) comprend, dans la zone de la cavité (AN), une couche partielle électriquement conductrice.

3. Capteur MEMS selon la revendication 2,
où la couche de recouvrement (DS) comprend une couche partielle en polysilicium.

4. Capteur MEMS selon l'une des revendications 1 à 3,
où la face supérieure de la couche de recouvrement (DS), dans la zone de la cavité (AN) et dans la zone limitrophe qui l'entoure, est située au même niveau au-dessus de la surface de la puce de base (BC).

5. Capteur MEMS selon l'une des revendications 2 à 4,
où la couche électriquement conductrice ou la couche partielle de l'électrode mobile (ME) et/ou de la couche de recouvrement (DS) est constituée de polysilicium.

6. Capteur MEMS selon l'une des revendications 1 à 5,
où la couche de recouvrement (DS) présente un motif d'ouvertures (OE) dans la zone de la cavité (AN).

7. Capteur MEMS selon l'une des revendications 1 à 6,
où la couche de recouvrement (DS) comprend une couche de nitrure de silicium.

8. Capteur MEMS selon la revendication 7,
où la couche de recouvrement (DS) comprend deux couches de nitrure de silicium entre lesquelles est disposée une couche de polysilicium (PS).

9. Capteur MEMS selon l'une des revendications 1 à 8,
où l'électrode mobile (ME) est constituée comme membrane et la couche de recouvrement (DS) ou une couche partielle de la couche de recouvrement présente, dans la zone limitrophe mais à l'intérieur de la cavité (AN), des excroissances en direction de la puce de base (BC), qui vont jusque directement au-dessus de la membrane.

10. Capteur MEMS selon l'une des revendications 1 à 9, où la membrane est disposée au-dessus d'une percée (DB) traversant toute l'épaisseur de la puce de base (BC).

11. Capteur MEMS selon l'une des revendications 1 à 9, où le matériau de couche sacrificielle comprend de l'oxyde de silicium.

12. Procédé de fabrication d'un capteur MEMS, comportant les étapes de procédé suivantes :
A. Application d'un système de couches (SA) à surface plane sur la surface d'une puce de base (BC), comprenant une série d'étapes de formation de couches et de structuration de couches, le système de couches comprenant une électrode mobile (ME) disposée en position centrale au-dessus de la puce de base, et qui est disposée entre deux couches sacrificielles (OS)
B. Gravure d'une tranchée (GR) dans le système de couches, qui entoure à distance l'électrode mobile et va jusqu'à la surface de la puce de base
C. Application d'une couche de recouvrement (DS) sur toute la surface du système de couches et dans la tranchée
D. Création d'un motif d'ouvertures (OE) dans la couche de recouvrement au-dessus de l'électrode mobile
E. Elimination des couches sacrificielles (OE) au-dessus et en-dessous de l'électrode mobile par gravure, la couche de recouvrement (DS) garnissant au moins la tranchée (GR) servant d'arrêt de gravure, où il se crée une cavité (AN) entourée par une zone limitrophe annulaire du système de couches, enjambée par la couche de recouvrement et dans laquelle est disposée l'électrode mobile.

13. Procédé selon la revendication 12,
où les couches sacrificielles (OS) comprennent de l'oxyde de silicium et où un agent de gravure contenant de l'hydrogène fluoré est utilisé pour l'élimination des couches sacrificielles.

14. Procédé selon la revendication 12 ou 13,
où au moins les étapes partielles suivantes sont réalisées pour créer la couche de recouvrement (DS) à l'étape C :
C1) Séparation d'une deuxième couche de polysilicium (PS2) recouvrant les bords
C2) Structuration de la deuxième couche de polysilicium en une électrode arrière
C3) Séparation et planarisation d'une couche d'oxyde C4) Séparation d'une première couche de nitrure de silicium (NS).

15. Procédé selon l'une des revendications 12 à 14,
où, à l'étape de procédé A, les étapes partielles suivantes sont effectuées :
A1) Création d'au moins une couche sacrificielle (OS) inférieure
A2) Création d'une première couche de polysilicium (PS) dopée par dessus
A3) Structuration de la première couche de polysilicium en une électrode mobile (ME)
A4) Création d'au moins une couche superficielle (OS) supérieure
A5) Planarisation du système de couches (SA).

16. Procédé selon la revendication 15,
- où, à l'étape A3), une première couche de polysilicium (PS1) est structurée en une membrane
- où, ensuite, des trous borgnes (SL) allant jusque dans la couche sacrificielle supérieure et des trous borgnes supplémentaires allant jusqu'à la membrane sont gravés dans la deuxième couche de polysilicium (PS2),
- où, à l'étape C4), la couche de nitrure de silicium (NS) est ainsi également séparée dans tous les trous borgnes en recouvrant les bords.

17. Procédé selon l'une des revendications 14 à 16,
où, après l'étape C3), à une étape C4), une couche de polysilicium (PS) supplémentaire est créée au-dessus de la couche de nitrure de silicium,
où, à une étape C5), une couche de nitrure de silicium (NS) supplémentaire est créée par dessus.

18. Procédé selon l'une des revendications 12 à 17,
où, avant l'étape E), une percée (DB) dans laquelle la couche sacrificielle (OS) inférieure est dégagée sous l'électrode mobile (ME) constituée comme membrane, est gravée dans la puce de base (BC) du côté arrière opposé au système de couches (SA).
